# EUROPEAN PATENT APPLICATION

(11) **EP 4 108 772 A1**
(43) Date of publication of application: **28.12.2022**
(21) Application number: 21757281.7
(22) Date of filing: 19.02.2021
(51) Int. Cl.: C12N 15/12, C12Q 1/686, C12Q 1/6876, C12N 9/00

(54) **METHOD FOR DETECTING TARGET NUCLEIC ACID USING DRIED BLOOD FILTER PAPER PIECE**

(30) Priority: 21.02.2020 JP 2020028303
(71) Applicant: Sekisui Medical Co., Ltd., Tokyo 103-0027 (JP)
(72) Inventor: EBINUMA Hiroyuki, Tokyo 103-0027 (JP); SUZUKI Ikumi, Tokyo 103-0027 (JP)
(74) Representative: Hoffmann Eitle
(86) International application number: PCT/JP2021/006319
(87) International publication number: WO 2021/167058

(57) **Abstract**

It is an object of the present disclosure to provide a method for reducing the influence of baseline disturbances in a method for detecting a target nucleic acid in dry blood filter paper by real-time PCR using a fluorescent dye, with a simple method. The present disclosure provides a method for detecting a target nucleic acid in dry blood filter paper by real-time PCR, the method including: (1) amplifying the target nucleic acid in the dry blood filter paper by applying thermal cycles to a sample solution containing a dry blood filter paper punch piece and a PCR reagent, wherein the PCR reagent includes a fluorescently labeled probe; (2) optically detecting the fluorescence intensity of the sample solution for each of the thermal cycles; and (3) performing quantitative analysis of the target nucleic acid using data after a predetermined number of cycles of the optically detected data.

## Description

### Technical Field

The present invention relates to a method for detecting a target nucleic acid by real-time PCR. More specifically, the present invention relates to a method for specifically detecting a target nucleic acid contained in dry blood filter paper in a method for detecting a target nucleic acid in dry blood filter paper by real-time PCR using a fluorescent dye by delaying the timing of fluorescence detection from the start of the PCR cycle.

### Background

In newborn screening tests using blood filter paper obtained by impregnating filter paper with blood, followed by drying (which may be hereinafter referred to as dry blood filter paper or simply as blood filter paper), as a sample, congenital diseases that have been attracting attention as genetic tests in recent years include primary immunodeficiency disease (PID) and spinal muscular atrophy (SMA).

Since the dry blood filter paper used for the tests contains large amounts of substances (such as hemoglobin) that inhibit the PCR amplification reaction, a method of extracting a nucleic acid from the blood filter paper and purifying it as a sample is generally used. Meanwhile, reagents that reduce the influence of PCR reaction inhibitors due to contaminants in the sample are also commercially available (Ampdirect (R), SHIMADZU CORPORATION).

PCR reaction can be performed directly from dry blood filter paper by using such reagents, and a technique for confirming the presence or absence of the target gene by detecting the PCR amplified product in the dry blood filter paper by electrophoresis has been disclosed (Patent Literature 1).

However, in the case where the PCR amplified product is detected using optical means, precipitation of blood components that occurs due to hemoglobin contained in blood and heat denaturation during PCR reaction obstructs the optical path, even if such a reagent is used, so that accurate results cannot be obtained. Therefore, a method for defining the addition ratio of a whole blood sample to a reaction solution during PCR amplification reaction has been disclosed (Patent Literature 2).

However, in addition to the influence derived from the whole blood components, the filter paper piece derived from dry blood filter paper present in the PCR reaction solution itself may obstruct the optical path for optical detection, and the influence may not be sufficiently reduced.

The dry blood filter paper is used to detect a target nucleic acid, where filter paper is dried after impregnated with blood, and taken out as punch hole-shaped sections (which may be referred to as dry blood filter paper punch piece in this description) by a punching tool. The inventors have found a method for amplifying a target nucleic acid directly from dry blood filter paper by real-time PCR and optically detecting and quantifying the amplified product by devising such as adjusting the size of a punch piece, the amount of whole blood contained in the punch piece, and the amount of a PCR reaction reagent to predetermined ranges, and attaching caps to PCR reaction tubes during PCR reaction without conventional complicated pretreatment, and have filed a patent application (Japanese Patent Application No. 2019-166510).

Meanwhile, even with such devising described above, contaminants contained in dry blood filter paper may cause baseline disturbances in the method for amplifying a target nucleic acid directly from dry blood filter paper by real-time PCR and optically detecting and quantifying the amplified product. This tendency is particularly high in the measurement system applying a fluorescent dye, which seems to be easily affected by hemoglobin contained in whole blood.

Here, a method for determining the baseline endpoint (termination cycle) of an amplification curve to prevent the deterioration in baseline quality caused by noisy data in real-time PCR has been proposed (Patent Literature 3). This method is a method of estimating the baseline endpoint by calculating the derivative of the amplification curve and processing the amplification curve by peak analysis of the primary derivative obtained. However, this method requires a new software to execute complex algorithms.

### Citation List

### Patent Literature

Patent Literature 1: Japanese Patent Laid-Open No. 2004-283165
Patent Literature 2: Japanese Patent No. 5144518
Patent Literature 3: Japanese Translation of PCT International Application Publication No. 2008-545380

### Summary of Invention

### Technical Problem

It is an object of the present invention to provide a method for reducing the influence of baseline disturbances with a simple method in a method for detecting a target nucleic acid in a dry blood filter paper punch piece by real-time PCR using a fluorescent dye.

### Solution to Problem

In the present invention, the inventors have found a method for accurately detecting and quantifying a target nucleic acid in dry blood filter paper without detecting baseline disturbances caused by contaminants contained in the dry blood filter paper for convenience by performing quantitative analysis of the target nucleic acid for the baseline disturbances, using data after a predetermined number of cycles of the optically detected data, such as by delaying the timing of fluorescence detection from the start of the PCR cycle, in a method for detecting a target nucleic acid in a dry blood filter paper punch piece by real-time PCR using a fluorescently labeled probe, thereby accomplishing the present invention.

That is, the present invention has the following configuration.
<1> A method for detecting a target nucleic acid in dry blood filter paper by real-time PCR, the method including:
   (1) amplifying the target nucleic acid in the dry blood filter paper by applying thermal cycles to a sample solution containing a dry blood filter paper punch piece and a PCR reagent, wherein the PCR reagent contains a fluorescently labeled probe;
   (2) optically detecting the fluorescence intensity of the sample solution for each of the thermal cycles; and
   (3) performing quantitative analysis of the target nucleic acid using data after a predetermined number of cycles of the optically detected data.
<2> The method according to <1>, wherein the predetermined number of cycles in (3) is 10 cycles or more and 25 cycles or less.
<3> The method according to <1> or <2>, wherein the data after the predetermined number of cycles in (3) is data obtained by optical detection started later than the start of the thermal cycles.
<4> The method according to any one of <1> to <3>, wherein the PCR reagent includes at least primers, polymerase, and dNTPs besides the fluorescently labeled probe.
<5> The method according to any one of <1> to <4>, wherein the fluorescently labeled probe has a fluorescent substance and a quencher and comprises a partial sequence complementary to the template of the nucleic acid amplification reaction, and the amplified target nucleic acid is detected by detection of the fluorescence generated by irradiation of the fluorescent substance with excitation light.
<6> The method according to any one of <1> to <5>, wherein the fluorescent label has a fluorescence detection wavelength in the range of 500 nm to 600 nm.
<7> The method according to any one of <1> to <6>, wherein the target nucleic acid is a nucleic acid of one or more genes selected from the group consisting of TREC, KREC, and SMN1.
<8> The method according to any one of <3> to <6>, wherein the step of optically detecting the fluorescence intensity of the sample solution for each of the thermal cycles in (2) is a step based on photometric parameters set in a thermal cycler and is a step in which optical detection is started later than the start of the thermal cycles by setting unphotometric parameters from the start of thermal cycles to the predetermined number of cycles and setting photometric parameters after the predetermined number of cycles.
<9> A method for reducing baseline disturbances in a method for detecting a target nucleic acid in dry blood filter paper by real-time PCR, the method including:
   (1) amplifying the target nucleic acid in the dry blood filter paper by applying thermal cycles to a sample solution containing a dry blood filter paper punch piece and a PCR reagent, wherein the PCR reagent includes a fluorescently labeled probe;
   (2) optically detecting the fluorescence intensity of the sample solution for each of the thermal cycles; and
   (3) performing quantitative analysis of the target nucleic acid using data after a predetermined number of cycles of the optically detected data.
<10> The method according to <9>, wherein the predetermined number of cycles in (3) is 10 cycles or more and 25 cycles or less.
<11> The method according to <9> or <10>, wherein the data after the predetermined number of cycles in (3) is data obtained by optical detection started later than the start of the thermal cycles.
<12> The method according to any one of <9> to <11>, wherein the PCR reagent includes at least primers, polymerase, and dNTPs besides the fluorescently labeled probe.
<13> The method according to any one of <9> to <12>, wherein the fluorescently labeled probe has a fluorescent substance and a quencher and comprises a partial sequence complementary to the template of the nucleic acid amplification reaction, and the amplified target nucleic acid is detected by detection of the fluorescence generated by irradiation of the fluorescent substance with excitation light.
<14> The method according to any one of <9> to <13>, wherein the fluorescent label has a fluorescence detection wavelength in the range of 500 nm to 600 nm.
<15> The method according to any one of <9> to <14>, wherein the target nucleic acid is a nucleic acid of one or more genes selected from the group consisting of TREC, KREC, and SMN1.
<16> The method according to any one of <11> to <15>, wherein the step of optically detecting the fluorescence intensity of the sample solution for each of the thermal cycles in (2) is a step based on photometric parameters set in a thermal cycler and is a step in which optical detection is started later than the start of the thermal cycles by setting unphotometric parameters from the start of thermal cycles to the predetermined number of cycles and setting photometric parameters after the predetermined number of cycles.

### Advantageous Effects of Invention

The present invention has enabled detection and quantification, while preventing erroneous determination due to poor baseline, in a method for directly subjecting dry blood filter paper to real-time PCR without pretreatment, amplifying a target nucleic acid in a dry blood filter paper punch piece by real-time PCR using a fluorescently labeled probe, and detecting the amplified product.

### Brief Description of Drawings

[Figure 1] Figure 1 is a graph showing an amplification curve when the RNaseP gene is amplified by real-time PCR of the present invention (Example 1).
[Figure 2] Figure 2 is a graph showing an amplification curve when the RNaseP gene is amplified by conventional real-time PCR (Comparative Example 1).

### Description of Embodiments

### (Dry blood filter paper)

The dry blood filter paper used in the present invention is blood (whole blood) in the filter paper obtained by impregnating the filter paper with whole blood, followed by drying, and may be simply referred to as dry blood filter paper. The dry blood filter paper is generally cut out from the filter paper impregnated with blood, followed by drying, and used as a paper section. Typically, it is taken out as a punch hole-shaped section (punch piece) with a punching tool to make holes. The punch piece-shaped filter paper section containing blood will be particularly referred to as a dry blood filter paper punch piece. The size of the punch piece used in the present invention is desirably such that the amount of blood contained in the reaction solution falls within a predetermined range, desirably a diameter of 1.2 mm to 2.0 mm, more desirably 1.5 mm to 1.8 mm. Further, the amount of whole blood contained in the punch piece is desirably 0.95 v/v% to 6.6 v/v%, more desirably 1.4 v/v% to 5.2 v/v%, with respect to the total amount of PCR reaction solution in terms of the size of the punch piece.

As the dry blood filter paper used in the present invention, for example, blood filter paper prepared from blood collected from the heel of a newborn baby such as newborn mass screening, and blood filter paper prepared from blood collected simultaneously as optional screening are used, but there is no limitation to these examples.

### (Target nucleic acid)

The target nucleic acid in the present invention is not particularly limited, and include such as DNAs and RNAs derived from whole blood, as well as foreign nucleic acids and the like introduced into whole blood by viruses and the like.

Among them, for example, nucleic acids derived from whole blood of newborns are preferable, and more specifically, include nucleic acids derived from genes such as TREC, KREC, SMN1, and SMN2 are included.

### (Real time PCR)

The PCR method is a widely used method, by repeating thermal cycles where temperature changes consisting of degeneration, annealing, and extension steps as one cycle, in the presence of the target nucleic acid, four types of deoxynucleoside triphosphates (dNTPs), a pair of primer sets, and polymerase, and exponentially amplifying the region of the target nucleic acid sandwiched between the primer sets. The number of thermal cycles appropriately varies depending on each reagent, the sample, and the reaction time, but is generally about 25 to 40 cycles.

In the present invention, the real-time PCR method means a method of monitoring the generation process of the amplified product of the target nucleic acid by the PCR method over time by optical means or the like.

In the present invention, optically detecting the fluorescence intensity of the sample solution for each of the thermal cycles means optically detecting the fluorescence intensity produced in the sample solution at the end of each of the thermal cycles.

The "step of performing quantitative analysis of the target nucleic acid using data after a predetermined number of cycles of the optically detected data", which is a feature of the present invention, roughly include the following two types:
(1) a step of performing analysis using the data obtained by the optical detection, wherein the optical detection is started later than the start of the thermal cycles; and
(2) a step of detecting the fluorescence intensity from the start of the thermal cycles but performing quantitative analysis of the target nucleic acid using data detected after a predetermined number of cycles without intentionally using data detected from the start of the thermal cycles to the predetermined number of cycles.

The sentence "optical detection is started later than the start of the thermal cycles" in (1) means that detection of the fluorescence intensity is not intentionally performed from the start of the thermal cycles to a predetermined number of cycles, and detection of the fluorescence intensity is performed from when the predetermined number of cycles are over, in the present invention, while optical detection has been conventionally started simultaneously with the start of the thermal cycles, and the fluorescence intensity has been detected from the end of the first cycle. The predetermined number of cycles may be the number of cycles before the baseline is stabilized and may be appropriately set depending on the type of the fluorescent dye to be used, the amount of hemoglobin in the sample, or the like.

The "step of detecting the fluorescence intensity from the start of the thermal cycles but performing quantitative analysis of the target nucleic acid using data detected after a predetermined number of cycles without intentionally using data detected from the start of the thermal cycles to the predetermined number of cycles" in (2) is generally performed after the completion of measurement by setting any cycle width and correcting the baseline.

The predetermined number of cycles is desirably 25 cycles at most and may be desirably 20 cycles or less. Further, the predetermined number of cycles is preferably at least 5 cycles or more and may be preferably 10 cycles or more. Therefore, the predetermined number of cycles in the present invention is preferably 5 cycles or more and 25 cycles or less and may be preferably 10 cycles or more and 25 cycles or less.

Conditions (temperature and time) for the thermal cycles in the PCR reaction of the present invention can be set as appropriate using a thermal cycler or the like, and the detection of the fluorescence intensity can be controlled as appropriate based on the photometric parameters set for each of the thermal cycles. In the present invention, unphotometric parameters are set from the start of thermal cycles to the predetermined number of cycles, and photometric parameters are set after the predetermined number of cycles, so that optical detection can be started later than the start of the thermal cycles. Accordingly, the step of optical detection by setting unphotometric parameters and subsequent photometric parameters after the predetermined cycles is also included in the step of "starting optical detection later than the start of the thermal cycles" of the present invention.

In the present invention, the amplified product of the target nucleic acid generated by PCR reaction can be detected by optically detecting the fluorescence intensity generated from the amplified product. The fluorescence detection of the present invention is achieved by generating fluorescence by irradiation of a fluorescent substance that is a fluorescently labeled probe with excitation light and optically detects the fluorescence, thereby enabling detection or quantification of the amplified target nucleic acid. The fluorescently labeled probe include TaqMan (R) probe, cycling probe, FRET probe and the like.

The detection of the fluorescence intensity can be performed, for example, using a fluorescence photometer. Further, a device provided with both a PCR reaction unit (such as a thermal cycler) and an optical system unit (such as a fluorescence photometer) is generally used. Specific examples thereof include commercially available SmartCycler (product name, Takara Bio Inc.), LightCycler (product name, Roche Diagnostics K.K.), ABI PRISM7000 (product name, Applied Biosystems), and the like.

### (PCR reagent)

The PCR reagent used in the present invention comprises a fluorescently labeled probe and further comprises at least a primer set, polymerase, and various dNTPs (deoxynucleoside triphosphates). The fluorescently labeled probe of the present invention comprises a fluorescent substance and a quencher, and desirably comprises a partial sequence complementary to the template of the nucleic acid amplification reaction.

The fluorescent dye for the fluorescently labeled probe may be any known fluorescent dye. Examples thereof include Alexa Fluor Series, CAL Fluor Series, Cy Series, ATTO Series, DY Series, DyLight Series, Quasar Series, FAM, TAMRA, TET, JOE, VIC, ROX, TexasRed, HEX, and the like, and it is particularly preferable to use a fluorescent dye with a fluorescence detection wavelength falling within the range of 500 to 600 nm since hemoglobin absorbs such a wavelength well, so that the effects of the present invention are exerted more. Examples thereof include FAM, TET, CAL Fluor(R) Gold540, JOE, VIC, HEX, CAL Fluor Orange560, Quasar(R), Cy^{™}3, NED, TAMRA, CAL Fluor Red590, and Cy3.5.

Further, the PCR reagent generally contains a buffer. The buffer is not specifically limited, as long as it is a buffer having a function of suppressing an action of a substance that inhibits DNA amplification reaction such as a positive charge substance (such as a type of protein) and a negative charge substance (such as a type of sugar or dye) that are present in the body fluid of an organism. Examples of commercially available products include Ampdirect, Ampdirect plus (both from SHIMADZU CORPORATION), and the like.

### (Quantification method of target nucleic acid)

The quantification method of the target nucleic acid of the present invention is a method of generating an amplified product complementary to a target nucleic acid in dry blood filter paper by real-time PCR, detecting the amplified product with an optical device, and quantifying the amplified product. The target nucleic acid contained in the dry blood filter paper can be quantified by counting the number of PCR cycles at which a predetermined amount of the amplified product has been produced. Further, as in Examples described later, it is also possible to convert the number of copies in 1 µL of whole blood in the dry blood filter paper from a calibration curve of Cq values calculated from a standard product, then quantify the target nucleic acid.

The method for detecting the target nucleic acid in the target nucleic acid in the dry blood filter paper by real-time PCR of the present invention includes the following steps (1) to (3):
(1) amplifying a target nucleic acid in dry blood filter paper by applying thermal cycles to a sample solution containing a dry blood filter paper punch piece and a PCR reagent, wherein the PCR reagent contains a fluorescently labeled probe;
(2) optically detecting the fluorescence intensity of the sample solution for each of the thermal cycles; and
(3) performing quantitative analysis of the target nucleic acid using data after a predetermined number of cycles of the optically detected data.

More specifically, step (1) above desirably includes the following steps (A) to (C):
(A) adding a dry blood filter paper to a PCR reaction tube;
(B) adding a PCR reagent to the PCR reaction tube, wherein the PCR reagent contains a fluorescently labeled probe; and
(C) amplifying the target nucleic acid in the dry blood filter paper by applying a plurality of thermal cycles to a sample solution containing a dry blood filter paper punch piece and a PCR reagent.

Here, any of step (A) and step (B) may be performed earlier, or the two may be performed simultaneously, but step (B) is desirably performed after step (A). This is because the filter paper is more reliably housed at the bottom part of the tube even if it adheres to the wall surface of the tube or the like due to the reagent solution by performing step (A) earlier. Further, in order to reliably house the punch piece at the bottom of the tube before starting the PCR reaction, centrifugation or the like is also desirably performed.

Further, it is preferable to start the thermal cycles after the reaction tube is sealed with a cap in the PCR reaction in the present invention, so that bubble formation due to the thermal cycles can be suppressed, and vibration can be suppressed without allowing the punch piece to float.

In the present invention, the phrase "applying thermal cycles" means to repeat temperature changes, and also to perform thermal cycling. Specifically, it also means to repeatedly giving temperature changes to the PCR reaction solution using a device called a thermal cycler.

The baseline disturbances can be reduced by step (3) described above of performing quantitative analysis of the target nucleic acid using data after a predetermined number of cycles of the optically detected data. In this regard, the present invention can be said to be, in other words, a method for reducing the baseline disturbances in the method for detecting a target nucleic acid in dry blood filter paper by real-time PCR.

The reason why the baseline disturbances are reduced is not clear, but it is inferred that some variations probably are caused in the baseline due to noise that is caused by contaminants contained in the dry blood filter paper and the degree at which the fluorescence produced from the fluorescently labeled probe due to PCR reaction is absorbed by the hemoglobin protein increasing or decreasing with the number of thermal cycles. The present invention seems to have enabled an accurate baseline to be drawn without being affected by the variation by not performing the fluorescence detection until the predetermined number of thermal cycles.

Accordingly, the present invention has enabled the target nucleic acid contained in the dry blood filter paper punch piece to be optically detected and quantified directly by real-time PCR without complicated pretreatment more accurately.

### (Reaction tube)

The tube used in the present invention is a PCR reaction tube and may have a structure that enables the dry blood filter paper punch piece to be smoothly inserted and sealed with a cap. Further, the opening in the upper part of the tube is preferably circular (for example, with an inner diameter of about 2.5 mm to 10 mm) and preferably has a shape with the bottom part being narrower than the opening in the upper part, such as an inverted cone shape.

The volume of the tube may be a volume capable of sufficiently housing the reaction solution by adding the PCR reagent and even with temperature changes due to the PCR and is preferably 0.1 mL to 0.3 mL, further preferably 0.15 mL to 0.25 mL, most preferably 0.2 mL.

The PCR reaction tube to be used in the present invention is preferably a 96-well tube for PCR reaction with consecutive 96 tubes or 8-series tube with consecutive 8 tubes, and those sold to the public under names such as 96-well PCR plates and 8-series PCR tubes can be used as commercially available products. The commercially available products include LightCycler 480 Multiwell Plate 96 (Roche Diagnostics K.K.), 96-Well PCR Plate, Flat Top, Low Profile, Natural, Polypropylene, UltraFlux (Scientific Specialties, Inc.), Eppendorf PCR plate 96, skirtless, 150 µL, colorless (Eppendorf AG), PCR plate 96-well thin plate 0.1-ml natural (BM Equipment Co., Ltd.), LightCycler(R) 8-Tube Strips (Roche Diagnostics K.K.), and the like.

Materials for these reaction tubes desirably have less contamination of the reaction solution and have a rigidity that does not allow the shape to be changed even due to internal pressure variation by the variation of the PCR reaction temperature change. Examples thereof include polypropylene.

Here, the amount of the PCR reagent used in the present invention to be added to the reaction tube is desirably 20 to 50 µL.

Hereinafter, the present invention will be described by way of examples, but is not limited to these examples.

### Examples

### [Example 1] Real-time PCR using fluorescently labeled probe

### (a) Test materials

### (a-1) Primers for RNaseP gene amplification

As primers for RNaseP amplification, the following primers were used on the request for synthesis to Sigma-Aldrich Co. LLC.
Forward primer: 5'-GCGGAGGGAAGCTCATCA-3' (SEQ ID No: 1)
Reverse primer: 5'-GTCTGACCTCGCGCGGA-3' (SEQ ID No: 2)

### (a-2) Probe for RNaseP gene detection

As a probe for confirming PCR amplification of RNaseP, the following fluorescently labeled (FAM) detection probe was used. The probe was created by requesting Primetech Corporation for synthesis. Probe for RNaseP gene detection: 5'-(FAM)-CCACGAGCTGAGTGCGTCCTG-(BHQ1)-3' (with a nucleotide sequence part represented by SEQ ID No: 3)

### (a-3)

As a template of the PCR reaction, a plasmid with the partial sequence of the RNaseP gene shown below being incorporated into a vector that was synthesized on request to Eurofins Scientific SE was used.

Partial sequence of RNaseP gene (SEQ ID No: 4)

CAACTTAATTTCTGATCATATTTTGTTGAATAAAATAAGTAAAATGTCTTGTGAAAC

AAAATGCTTTTTAACATCCATATAAAGCTATCTATATATAGCTATCTATGTCTATAT

AGCTATTTTTTTTAACTTCCTTTATTTTCCTTACAGGGTTTCAGACAAAATCAAAAA

GAAGGAAGGTGCTCACATTCCTTAAATTAAGGAGTAAGTCTGCCAGCATTATGAAAG

TGAATCTTACTTTTGTAAAACTTTATGGTTTGTGGAAAACAAATGTTTTTGAACATT

TAAAAAGTTCAGATGTTAAAAAGTTGAAAGGTTAATGTAAAACAATCAATATTAAAG

AATTTTGATGCCAAAACTATTAGATAAAAGGTTAATCTACATCCCTACTAGAATTCT

C

### (b) PCR reagent

The composition of the PCR reagent is shown below. PCR buffer (Ampdirect Plus; SHIMADZU CORPORATION)
0.125 µM (final concentration) forward primer for RNaseP gene amplification
0.125 µM (final concentration) reverse primer for RNaseP gene amplification
0.125 µM (final concentration) probe for RNaseP gene detection
0.025 U/µL BIOTAQ HSDNA polymerase

### (c) Control dry blood filter paper

To blood obtained by mixing human erythrocyte fraction with leukocytes removed and 1% BSA (PBS base) at a ratio of 6:4, were added plasmids containing the partial sequence of the RNaseP gene to 10,000 copies/µL, and filter paper for collecting blood (ADVANTEC CO., LTD.) was impregnated with 40 µL thereof, followed by drying as it was, which was used as a control dry blood filter paper.

### (d) Conditions for PCR reaction

The PCR reaction was performed under conditions combining the unphotometric parameters of the present invention shown below.
(i) At 95°C for 15 minutes
(ii) At 95°C for 15 seconds and at 63°C for 60 seconds (10 cycles) with unphotometric setting
(iii) At 95°C for 15 seconds and at 63°C for 60 seconds (30 cycles) with photometric setting
(iv) At 37°C for 5 minutes

### (e) Real-time PCR measurement

### (e-1) Measurement method

The measurement was performed by the following procedure.
(i) A circular punch piece with a diameter of 1.5 mm was collected from the control dry blood filter paper using a puncher.
(ii) The punch piece was put into a PCR reaction tube (LightCycler(R) 8-Tube Strips, made of polypropylene and available from Roche Diagnostics K.K.).
(iii) After 40 µL of the PCR reagent was added to each tube, it was sealed with a cap (set with the tube).
(iv) The real-time PCR measurement for RNaseP gene (excitation: 470 nm/detection: 514 nm) was performed with the aforementioned parameters using a thermal cycler device (LightCycler 96, available from Roche Diagnostics

### K.K.).

### (e-2) Measurement results

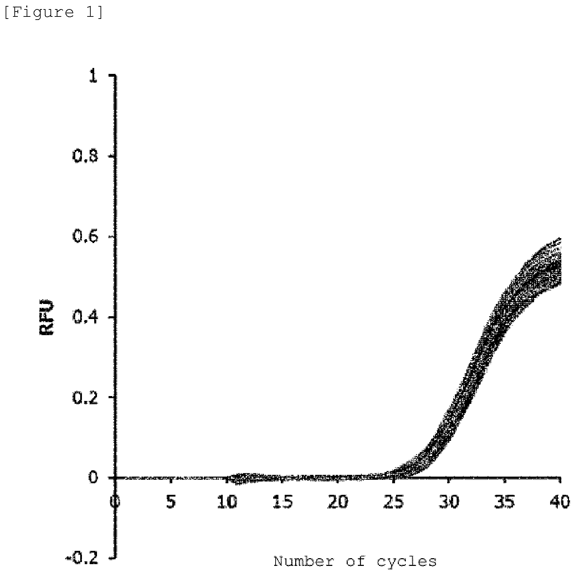

Fig. 1 shows the amplification curves of PCR reaction as a result of measurement under the PCR reaction conditions of the present invention. As a result of measuring each of the 20 punch pieces by combining unphotometric parameters, it was confirmed that baseline correction substantially free from disturbances as generated in Comparative Example 1, which will be described below, was performed well.

Thus, parameters that do not detect fluorescence were incorporated into the baseline disturbances caused by contaminants contained in the dry blood filter paper, thereby enabling the setting such that the baseline disturbances were, for convenience, not detected. Accordingly, the method of the present invention could provide an accurate and efficient quantification method that suppresses the frequency of re-examinations due to poor baseline when measuring a specific gene in dry blood filter paper.

### [Comparative Example 1] Real-time PCR with conventional parameters using fluorescently labeled probe

### (a) Real-time PCR measurement

Real-time PCR was performed using a FAM-labeled fluorescence probe by the same method except that (d) the parameters of the conditions for PCR reaction in Example 1 above were changed as follows, to measure the RNaseP gene of each of the 44 punch pieces.

### <PCR reaction conditions: Conventional parameters>

(i) At 95°C for 15 minutes
(ii) At 95°C for 15 seconds and 63°C for 60 seconds (40 cycles) with photometric setting
(iii) At 37°C for 5 minutes

### (b) Measurement results

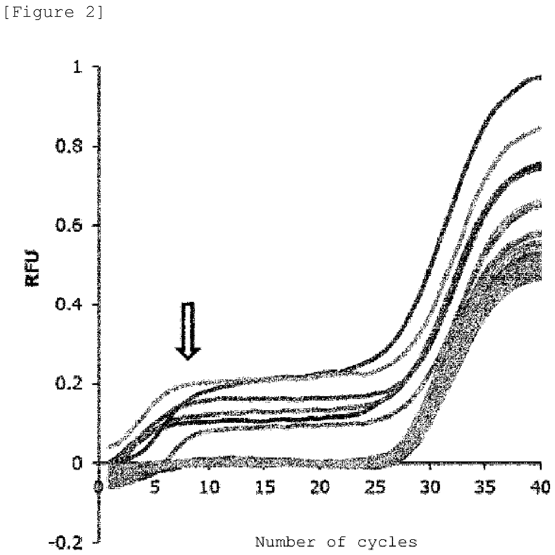

Fig. 2 shows amplification curves of PCR reaction measured for 44 punch pieces.

In 6 out of 44 amplification curves, baseline disturbances were seen even after the baseline correction function was built in the device. Although the reason is not clear, it may be related to the amount of hemoglobin protein eluted from the sample, i.e. dry blood filter paper punch pieces. Namely, there may be cases where the fluorescence value temporarily became relatively low due to the fluorescence of FAM as a fluorescent substance being absorbed by a protein at the initial stage of the reaction, and the influence of hemoglobin protein was weakened as the number of thermal cycles progresses. In such a case, the fluorescence value increases transiently, and a computation unit of the device processes it as if the amplification reaction occurred at the time point (the number of cycles) of the arrow part of the growth curve, so that the Cq value was calculated to be very small.

Accordingly, when fitting to the calibration curve is performed based on the Cq value, the concentration of the target nucleic acid in the sample will be erroneously calculated to be much higher than the actual concentration.

### Industrial Applicability

The present invention enabled accurate quantification by performing quantitative analysis of the target nucleic acid using data after a predetermined number of cycles of the optically detected data, such as delaying the timing of fluorescence detection to be later than the start of PCR thermal cycles in a method for detecting a target nucleic acid in a dry blood filter paper punch piece by real-time PCR using a fluorescently labeled probe, to prevent erroneous determination due to poor baseline.

The method of the present invention can be applied to quantification of specific genes, such as the TREC/KREC gene fragment quantification method as a newborn screening test of PID and the SMN1 gene quantification method as a newborn screening test of SMA.

## Claims

1. A method for detecting a target nucleic acid in dry blood filter paper by real-time PCR, the method comprising:
(1) amplifying the target nucleic acid in the dry blood filter paper by applying thermal cycles to a sample solution containing a dry blood filter paper punch piece and a PCR reagent, wherein the PCR reagent contains a fluorescently labeled probe;
(2) optically detecting the fluorescence intensity of the sample solution for each of the thermal cycles; and
(3) performing quantitative analysis of the target nucleic acid using data after a predetermined number of cycles of the optically detected data.

2. The method according to claim 1, wherein the predetermined number of cycles in (3) is 10 cycles or more and 25 cycles or less.

3. The method according to claim 1 or 2, wherein the data after the predetermined number of cycles in (3) is data obtained by optical detection started later than the start of the thermal cycles.

4. The method according to any one of claims 1 to 3, wherein the PCR reagent comprises at least primers, polymerase, and dNTPs besides the fluorescently labeled probe.

5. The method according to any one of claims 1 to 4, wherein the fluorescently labeled probe comprises a fluorescent substance and a quencher and comprises a partial sequence complementary to the template of the nucleic acid amplification reaction, and the amplified target nucleic acid is detected by detection of the fluorescence generated by irradiation of the fluorescent substance with excitation light.

6. The method according to any one of claims 1 to 5, wherein the fluorescent label has a fluorescence detection wavelength in the range of 500 nm to 600 nm.

7. The method according to any one of claims 1 to 6, wherein the target nucleic acid is a nucleic acid of one or more genes selected from the group consisting of TREC, KREC, and SMN1.

8. The method according to any one of claims 3 to 6, wherein the step of optically detecting the fluorescence intensity of the sample solution for each of the thermal cycles in (2) is a step based on photometric parameters set in a thermal cycler and is a step in which optical detection is started later than the start of the thermal cycles by setting unphotometric parameters from the start of thermal cycles to the predetermined number of cycles and setting photometric parameters after the predetermined number of cycles.

9. A method for reducing baseline disturbances in a method for detecting a target nucleic acid in dry blood filter paper by real-time PCR, the method comprising:
(1) amplifying the target nucleic acid in the dry blood filter paper by applying thermal cycles to a sample solution containing a dry blood filter paper punch piece and a PCR reagent, wherein the PCR reagent comprises a fluorescently labeled probe;
(2) optically detecting the fluorescence intensity of the sample solution for each of the thermal cycles; and
(3) performing quantitative analysis of the target nucleic acid using data after a predetermined number of cycles of the optically detected data.

10. The method according to claim 9, wherein the predetermined number of cycles in (3) is 10 cycles or more and 25 cycles or less.

11. The method according to claim 9 or 10, wherein the data after the predetermined number of cycles in (3) is data obtained by optical detection started later than the start of the thermal cycles.

12. The method according to any one of claims 9 to 11, wherein the PCR reagent comprises at least primers, polymerase, and dNTPs besides the fluorescently labeled probe.

13. The method according to any one of claims 9 to 12, wherein the fluorescently labeled probe comprises a fluorescent substance and a quencher and comprises a partial sequence complementary to the template of the nucleic acid amplification reaction, and the amplified target nucleic acid is detected by detection of the fluorescence generated by irradiation of the fluorescent substance with excitation light.

14. The method according to any one of claims 9 to 13, wherein the fluorescent label has a fluorescence detection wavelength in the range of 500 nm to 600 nm.

15. The method according to any one of claims 9 to 14, wherein the target nucleic acid is a nucleic acid of one or more genes selected from the group consisting of TREC, KREC, and SMN1.

16. The method according to any one of claims 11 to 15, wherein the step of optically detecting the fluorescence intensity of the sample solution for each of the thermal cycles in (2) is a step based on photometric parameters set in a thermal cycler and is a step in which optical detection is started later than the start of the thermal cycles by setting unphotometric parameters from the start of thermal cycles to the predetermined number of cycles and setting photometric parameters after the predetermined number of cycles.
